# EUROPÄISCHE PATENTANMELDUNG

(11) **EP 2 133 045 A2**
(43) Veröffentlichungstag der Anmeldung: **16.12.2009**
(21) Anmeldenummer: 09160150.0
(22) Anmeldetag: 13.05.2009
(51) Int. Cl.: A61F 2/86

(54) **Implantierbare Vorrichtung**

(30) Priorität: 12.06.2008 DE 102008002397
(71) Anmelder: Biotronik VI Patent AG, 6341 Baar (CH)
(72) Erfinder: Tittelbach, Michael, 90429, Nürnberg (DE); Fargahi, Amir, 5242, Birr (CH)
(74) Vertreter: Lindner-Vogt, Karin L.

(57) **Zusammenfassung**

Die Erfindung betrifft eine implantierbare Vorrichtung zur lokalen Abgabe wenigstens eines Wirkstoffs (11) an vorgegebener Stelle in einen natürlichen Hohlraum (12) mit einer Wand (13) im Körper eines Säugers, gekennzeichnet durch wenigstens ein Wirkstoffreservoir (14), das in dem Hohlraum (12) fixierbar ist, wobei das Wirkstoffreservoir (14) und/oder ein Wirkstoffausgang (15) beabstandet zur Wand (13) so angeordnet ist, dass eine Freisetzung des wenigstens einen Wirkstoffs (11) entfernt zur Wand (13) erfolgt.

## Beschreibung

Die Erfindung betrifft eine implantierbare Vorrichtung nach den Oberbegriffen der unabhängigen Ansprüche.

In der Medizintechnik sind implantierbare Vorrichtungen zur lokalen Wirkstoffabgabe an vorgegebener Stelle in einen natürlichen Hohlraum eines Säugers, beispielsweise in einen Gewebekanal, vielfach bekannt. Die bestehenden Lösungsvorschläge sind häufig nicht in der Lage, ausreichend große Mengen an Wirkstoff in definierte Regionen einzubringen. Schwierigkeiten ergeben sich oft beim Einsatz von beispielsweise zwei oder mehreren verschiedenen Wirkstoffen zur gleichen Zeit. Andere Lösungsvorschläge betreffen einen voll degradierbaren Gefäßstützkörper mit hoher Wirkstoffbeladung. Problematisch dabei ist, dass derartige Gefäßstützkörper durch direkten Kontakt der Wirkstoff freisetzenden Implantatteile mit der Gefäßwand erhebliche Nebenwirkungen lokal im Gewebe der Gefäßwand hervorrufen können, insbesondere bei der Abgabe von zytostatischen Wirkstoffen. Derartige Nebenwirkungen können aufwendige Therapien zur Prophylaxe und Minderung der Nebenwirkungen zur Folge haben.

Der Erfindung liegt die Aufgabe zugrunde, eine implantierbare Vorrichtung bereitzustellen, die in der Lage ist, große Mengen eines oder mehrerer Wirkstoffe in einen natürlichen Hohlraum eines Säugers, beispielsweise in einen Gewebekanal, insbesondere in ein arterielles Gefäß, abzugeben, ohne dass dabei die Wand des Hohlraums im Bereich der implantierbaren Vorrichtung mit dem Wirkstoff belastet wird.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Bei einer erfindungsgemäßen implantierbaren Vorrichtung ist wenigstens ein Wirkstoffreservoir vorgesehen, wobei das Wirkstoffreservoir in dem Hohlraum fixierbar ist, und wobei das Wirkstoffreservoir und/oder ein Wirkstoffausgang beabstandet zur Wand so angeordnet ist, dass eine Freisetzung des Wirkstoffs entfernt von der Wand erfolgt. Eine alternative erfindungsgemäße implantierbare Vorrichtung betrifft ein Wirkstoffreservoir mit einem Wirkstoffausgang, wobei der Wirkstoffausgang durch eine perforierte Hülle einer spiralförmig oder schraubenförmig gewundenen Spule gebildet ist. Vorteilhafterweise erlauben diese Lösungsvorschläge eine Implantation von großen Wirkstoffmengen in voraus bestimmte Regionen eines Hohlraums, insbesondere von Arterien, ohne die Gefäßwand mit dem Wirkstoff zu belasten. Günstigerweise können somit unerwünschte Nebenwirkungen wie Entzündungen oder Nekrosen vermieden werden. Nachfolgend wird der Hohlraum nur noch mit Arterie bzw. arteriellem Gefäß bezeichnet.

Die implantierbare Vorrichtung wird durch ein vorbekanntes Einführsystem (SDS) mittels eines Katheters in das arterielle Gefäß eingebracht. Bevorzugt ist das Implantat als implantierbarer Stützkörper ausgebildet, beispielsweise als Stent, der in das arterielle Gefäß eingebracht wird. In einer Variante kann als implantierbarer Stützkörper auch eine Struktur aus einem schrauben- oder spiralförmigen Röhrchen (Coil) vorgesehen sein, das in das arterielle Gefäß eingesetzt wird. Bevorzugt ist der implantierbare Stützkörper aus Nitinol gebildet. Die implantierbaren Stützkörper können günstigerweise als Fixierelement für ein Wirkstoffreservoir dienen, wobei besonders bevorzugt der Wirkstoffausgang zentral in der Arterie angeordnet ist. Eine ungünstige Belastung der Gefäßwand mit abgegebenen Wirkstoffen kann dabei vermieden werden. Wird mehr als ein Wirkstoff zur Therapie benötigt, kann das Wirkstoffreservoir aus mehreren Wirkstoffbehältern bestehen.

Das Wirkstoffreservoir kann auch aus mehreren Teilen mit verschiedenen Wirkstoffen bestehen. Das Wirkstoffreservoir weist bevorzugt an wenigstens einem Ende das besonders bevorzugt als Stent und/oder als Spule ausgebildete Fixierelement auf, damit der Wirkstoffausgang in einer definierten Position innerhalb des Gefäßes gehalten und gegen die Hohlraumwände gestützt wird. Das Wirkstoffreservoir kann bevorzugt aus der Spule selbst mit einem proximalen und einem distalen Ende gebildet sein, wobei das proximale Ende an der Innenwand des Gefäßes anliegt. Das proximale Ende der Spule ist dabei günstigerweise so ausgeführt, dass er ohne Beeinflussung der Hämodynamik an der Gefäßwand anliegt. Mit seinem distalen Ende ist die Spule so ausgeführt, dass sie etwa mittig in das arterielle Gefäß ragt, wobei das distale Ende zur Wirkstoffabgabe per Diffusion vorgesehen ist. Je mittiger das distale Ende angeordnet ist, umso vorteilhafter ist es.

Besonders bevorzugt ist das Wirkstoffreservoir an seinen beiden Enden mit Hilfe der Fixierelemente gehalten, vorzugsweise koaxial fixiert. Günstigerweise wird das Wirkstoffreservoir dabei entweder einseitig oder zweiseitig durch Spulen oder stentartige Stützkörper zentral in der Blutbahn gehaltert. Besonders bevorzugt sind die Spulen und/oder die Stents selbst expandierend und justieren sich somit selbst an einer definierten Stelle in dem Gefäß.

In einer vorteilhaften Variante kann das Fixierelement selbst den Wirkstoffausgang bilden. Als Wirkstoffreservoir können dabei die Spule oder der Stent selbst dienen, welche besonders bevorzugt eine poröse Hülle aufweisen. Die Wirkstoffabgabe kann dabei per Diffusion über eine kapillare Wirkung erfolgen, wobei wiederum eine lokale Belastung der inneren Gefäßwand durch den abgegebenen Wirkstoff günstigerweise vermieden werden kann. Der Wirkstoff kann dabei vorteilhafterweise mit Hilfe der Kapillarwirkung aus dem Wirkstoffreservoir transportiert und in der Blutbahn freigesetzt werden, ohne dass die Gefäßwand unmittelbar belastet wird.

In einer weiteren vorteilhaften Variante kann die Wirkstoffabgabe über biologisch abbaubare Materialien erfolgen. Bevorzugt ist das Material aus einem bioabbaubaren Polymer oder aus einem Metall, beispielsweise aus Edelstahl und Tantal, Platin und Nitinol, gebildet. Günstigerweise ist das Wirkstoffreservoir bei dieser Variante aus einem mehrschichtigen Körper aus vorzugsweise biologisch abbaubaren Materialien gebildet, wobei die Räume zwischen den Schichten mit demselben oder mit unterschiedlichen Wirkstoffen ausgefüllt sind. Vorteilhafterweise kann der Wirkstoff nach Abbau der Schichten freigegeben werden, wobei eine ungünstige Belastung der Gefäßwand wiederum entfällt. Sind die Zwischenräume mit demselben Wirkstoff gefüllt, kann die Wirkstoffabgabe günstigerweise gesteuert ablaufen, indem der Wirkstoff über eine ausreichend lange Zeit zur Verfügung gestellt wird. Alternativ oder zusätzlich kann auch eine primär diffusionsgesteuerte Freisetzung eines oder mehrerer Wirkstoffe aus dem biologisch abbaubaren Material erfolgen; als Wirkstoffausgang kann dann die Oberfläche des Körpers dienen.

In einer weiteren Variante kann das Wirkstoffreservoir aus einem bevorzugt stromlinienförmigen Körper aus einem Polymer gebildet sein, der mit dem gewünschten Wirkstoff beladen ist. Durch die geometrische Form kann der Polymerkörper eine Selbstjustierung des Wirkstoff freisetzenden Implantatteils in der Gefäßmitte bewirken. Der Körper kann weitere Fixierflügel aufweisen, wobei die Fixierflügel kleine Kontaktflächen zur Gefäßwand aufweisen und für eine lumenerhaltende Aufhängung des Implantats im Gefäß sorgen. Die Kontaktfläche ist bevorzugt linienförmig, besonders bevorzugt punktförmig ausgebildet. Vorteilhafterweise ist dabei allenfalls ein geringer Wirkstoffeintrag in die Gefäßwand zu verzeichnen. Alternativ können die Fixierflügel wirkstofffrei ausgeführt sein.

Die einzelnen Varianten und Ausführungsformen können auch beliebig miteinander kombiniert werden. Bei allen Ausführungsvarianten können vorteilhafterweise große Mengen, beispielsweise 5 mg bis 10 mg, eines oder mehrerer Wirkstoffe in ein arterielles Gefäß abgegeben werden, ohne dass dabei die Gefäßwand im Bereich des Implantats mit dem Wirkstoff belastet wird. Unerwünschte Nebenwirkungen können somit vorteilhafterweise vermieden werden. Bei einer besonders bevorzugten Anwendung kann die erfindungsgemäße implantierbare Vorrichtung in ein arterielles Gefäß kurz vor einem Organ, z.B. der Leber, der Niere etc., eingebracht sein, wobei günstigerweise eine große Menge an Wirkstoff in großem Stromfluss in das Gefäß einbringbar ist, wodurch der Wirkstoff schnell ans Zielgebiet abgegeben wird.

Die Erfindung ist nachfolgend beispielhaft, anhand von in Zeichnungen dargestellten Ausführungsbeispielen, näher erläutert. Es zeigen in schematischer Darstellung:
- Fig. 1: einen Schnitt durch einen als arterielles Gefäß ausgebildeten Hohlraum mit einer bevorzugten Ausführungsform einer implantierbaren Vorrich- tung zur lokalen Abgabe wenigstens eines Wirkstoffs an einer vorgege- benen Stelle in das Gefäß, wobei die implantierbare Vorrichtung aus ei- nem Wirkstoffreservoir gebildet ist, das an beiden Enden durch einen jeweils als Spule ausgebildeten Stützkörper fixiert ist;
- Fig. 2: einen Schnitt durch das arterielle Gefäß mit einer weiteren bevorzugten Variante gemäß einem weiteren, unabhängigen Aspekt der Erfindung
- Fig. 3: eine Schnittdarstellung durch das arterielle Gefäß mit einer bevorzugten alternativen implantierbaren Vorrichtung, die aus einem Wirkstoffreser- voir und einer Spule mit perforierter Hülle gebildet ist;
- Fig. 4 bis Fig. 9: weitere Varianten einer implantierbaren Vorrichtung; und
- Fig. 10a, 10b: eine alternative bevorzugte Ausführungsform einer als im arteriellen Ge- fäß selbstjustierender Polymerkörper ausgebildeten implantierbaren Vor- richtung in einem Querschnitt (Fig. 10a) und in einem Längsschnitt (Fig. 10b).

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

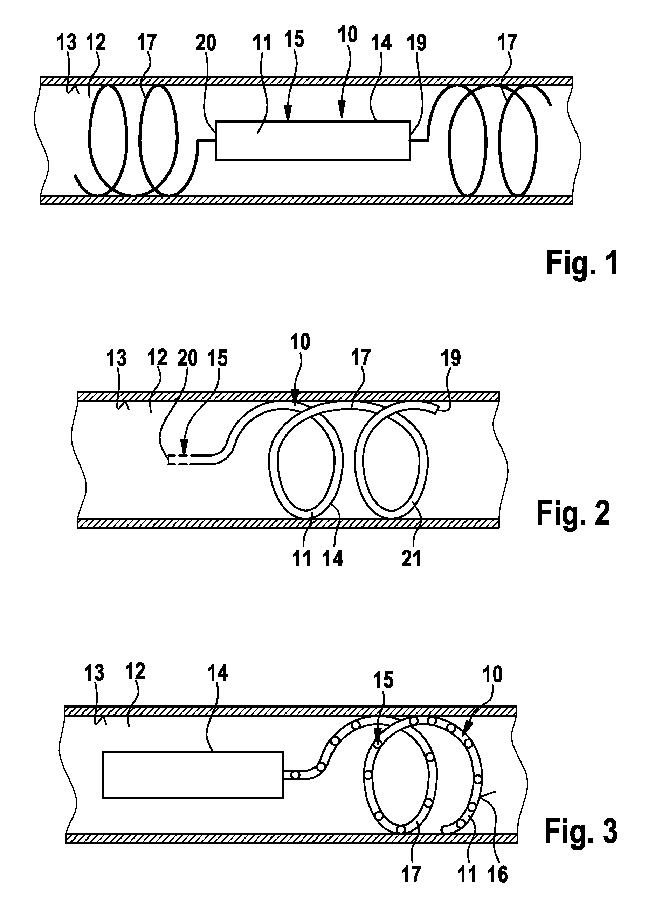

Fig. 1 zeigt in einer schematischen Darstellung einen Längsschnitt durch einen als arterielles Gefäß 12 ausgebildeten Hohlraum mit einer bevorzugten Ausführungsform eines Implantats 10 zur lokalen Abgabe wenigstens eines Wirkstoffs 11 an einer vorgegebenen Stelle in das Gefäß 12. Das Implantat 10 umfasst ein Wirkstoffreservoir 14, das an seinen beiden Enden 19 und 20 mit zwei als Spule 17 ausgebildeten Stützkörpern verbunden ist. Die Spule 17 ist jeweils ein schrauben- oder spiralförmig gewundenes Röhrchen, das so in dem Gefäß 12 angeordnet ist, dass es koaxial zu dem Gefäß 12 sowie zu dem Wirkstoffreservoir 14 angeordnet ist. Besonders bevorzugt sind die Spulen 17 selbst expandierend und fixieren sich somit selbsttätig nach dem Einbringen in das Gefäß 12 dermaßen, dass die Spulen 17 an ihrer Peripherie mit der Gefäßinnenwand 13 in Kontakt stehen. Das Wirkstoffreservoir 14 ist mit wenigstens einem Wirkstoff 11 befüllt, der per Diffusion durch Kapillarwirkung in das Gefäß 12 eingebracht wird. Der Wirkstoffausgang 15 wird durch die Oberfläche des Wirkstoffreservoirs 14 gebildet. Insbesondere ist das Wirkstoffreservoir 14 so in dem Gefäß 12 fixiert, dass das Wirkstoffreservoir 14 und somit der Wirkstoffausgang 15 beabstandet zur Gefäßwand 13 angeordnet ist. Vorteilhafterweise wird somit eine Belastung der Gefäßwand 13 und/oder des Lumens mit dem Wirkstoff 11 vermieden.

In Fig. 2 ist eine weitere bevorzugte Variante gemäß einem weiteren, unabhängigen Aspekt der Erfindung gezeigt, wobei die implantierbare Vorrichtung 10 als Spule 17 ausgebildet ist, die in ein arterielles Gefäß 12 eingebracht ist. Die Spule 17 dient als Fixierelement 21 und bildet gleichzeitig das Wirkstoffreservoir 14 mit einem Wirkstoffausgang 15. Die Spule 17 weist ein proximales Ende 19 und ein distales Ende 20 aus, wobei das proximale Ende 19 im Bereich der Innenwand 13 des arteriellen Gefäßes 12 angeordnet ist. Mit ihrem distalen Ende 20 ist die Spule 17 etwa mittig im Hohlraum 12 angeordnet. Das distale Ende 20 bildet den Wirkstoffausgang 15, der zentral im Gefäß 12 angeordnet ist.

In Fig. 3 ist eine weitere Variante einer in ein arterielles Gefäß 12 implantierbaren Vorrichtung 10 dargestellt, welche ein mit einer Spule 17 verbundenen Wirkstoffreservoir 14 umfasst. Der Wirkstoffausgang 15 ist durch eine poröse, insbesondere perforierte Hülle 16 der Spule 17 gebildet. Der Wirkstoff 11 diffundiert über die perforierte Hülle 16 der Spule 17 in das Gefäß 12, ohne dabei die Wand 13 des Gefäßes 12 lokal mit Wirkstoff 11 zu belasten.

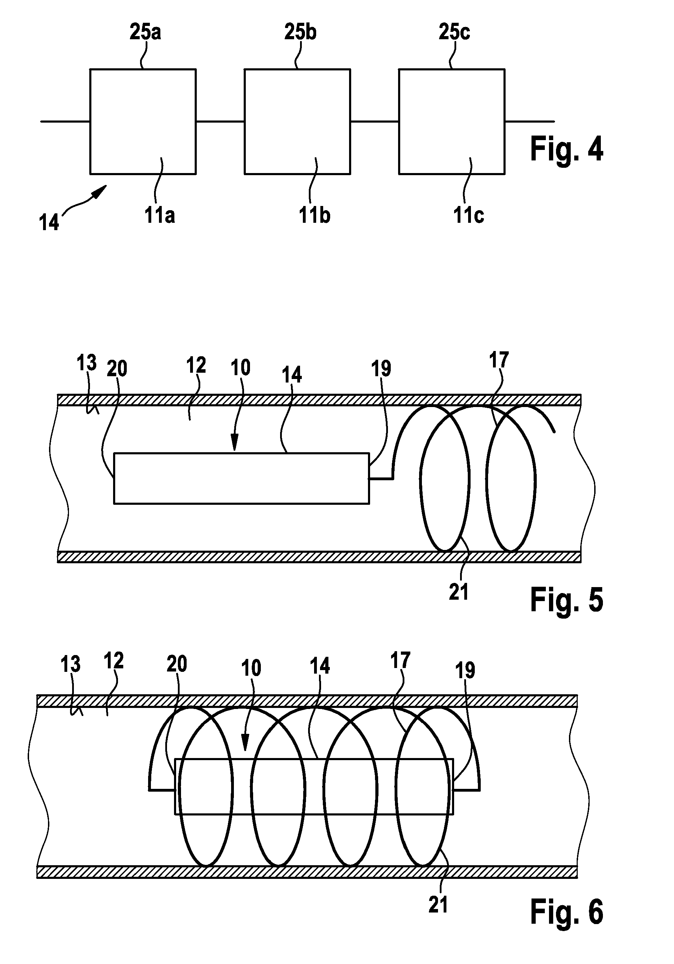

Wie in Fig. 4 schematisch dargestellt ist, kann das Wirkstoffreservoir 14 aus mehreren Wirkstoffbehältern 25a, 25b, 25c bestehen, in welche jeweils verschiedene Wirkstoffe 11a, 11b, 11c eingebracht sind. Diese können in das Gefäß 12 eingebracht werden, wie in den übrigen Figuren beschrieben ist.

Die in Fig. 5 dargestellte implantierbare Vorrichtung 10 ist aus einem Wirkstoffreservoir 14 gebildet, das an einem seiner Enden 19, 20 mit einer Spule 17 verbunden ist. Die Spule 17 ist bevorzugt selbst expandierend ausgebildet und stützt sich in einer Endposition an der Innenwand 13 des Gefäßes 12 ab und wird dadurch fixiert. Die Spule 17 dient dabei als Fixierelement 21 für das Wirkstoffreservoir 14, das besonders bevorzugt zentral im Gefäß 12 gehalten wird.

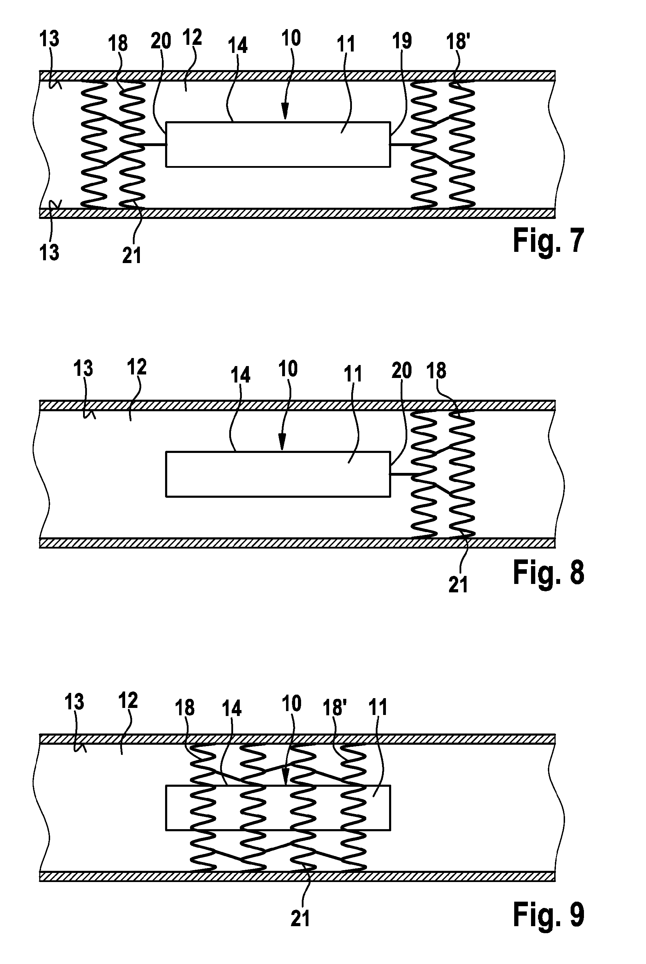

In Fig. 6 ist das Wirkstoffreservoir 14 so mit einer Spule 17 verbunden, dass es koaxial zur Spule 17 in dem Gefäß 12 fixiert wird. Das Wirkstoffreservoir 14 ist dabei als länglicher, zylindrischer Körper ausgebildet, der an seinen beiden Enden 19, 20 mit der Spule 17 verbunden und koaxial innerhalb der Spule 17 angeordnet ist. Fig. 7 zeigt eine weitere Variante eines bevorzugten implantierbaren Körpers 10, wobei ein Wirkstoffreservoir 14 an beiden Enden 19, 20 ein als Stent 18, 18' ausgebildetes Fixierelement 21 aufweist. Günstigerweise sind die Stents 18, 18' selbst expandierend und fixieren sich selbsttätig in einer definierten Endposition in dem arteriellen Gefäß 12. In einer alternativen Ausführungsform gemäß Fig. 8 ist das Wirkstoffreservoir 14 an einem bevorzugt distalen Ende 20 mit einem Stent 18 verbunden. Der Stent 18 dient als Fixierelement 21 für das Wirkstoffreservoir 14, das in einem zentralen Bereich des Gefäßes 12 gehalten wird. In einer weiteren Variante gemäß Fig. 9 ist ein Wirkstoffreservoir 14 mit Hilfe eines als Stent 18, 18' ausgebildeten Fixierelements 21 koaxial fixiert. Der in das Wirkstoffreservoir 14 eingebrachte Wirkstoff 11 kann jeweils per Diffusion durch Kapillarwirkung in das Gefäß 12 freigesetzt werden, ohne die Gefäßwand 13 oder das Lumen zu beeinträchtigen. In einer Variante kann der Wirkstoff 11 durch Degradation einer Hülle des Wirkstoffreservoirs 14 bildenden, biologisch abbaubare Materialien, in das Gefäß 12 freigesetzt werden. Als biologisch abbaubare Materialien werden beispielsweise Polymer oder Metall verwendet.

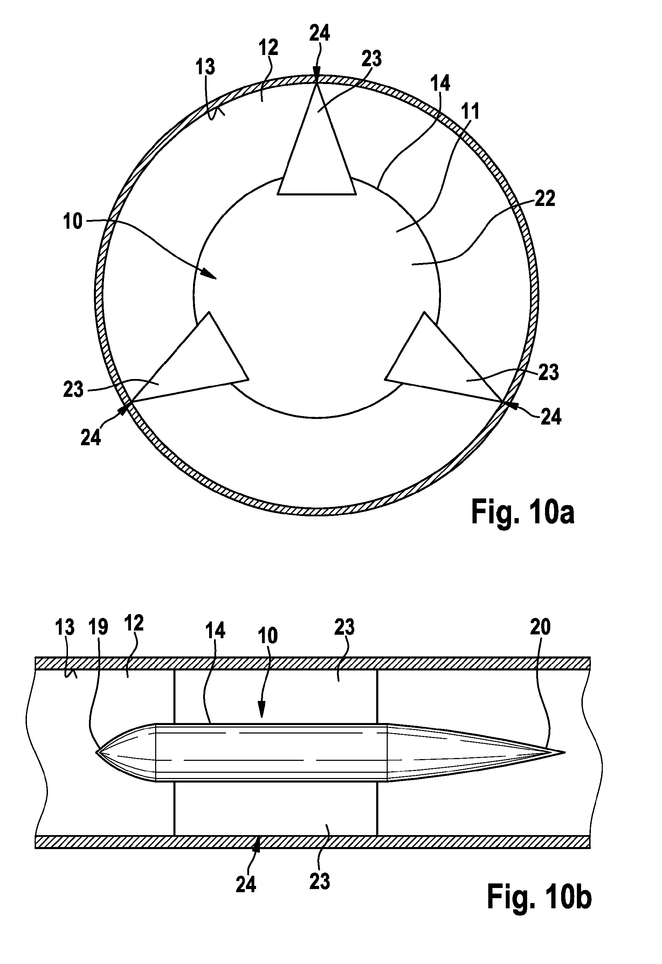

In einer besonders bevorzugten Variante gemäß Fig. 10a, 10b ist das Wirkstoffreservoir 14 aus einem mehrschichtigen Polymerkörper 22 aus biologisch abbaubaren Materialien gebildet, wobei Räume zwischen den im einzelnen nicht näher dargestellten Schichten mit dem wenigstens einen Wirkstoff 11 gefüllt sind. Nach Abbau der Schichten kann der Wirkstoff 11 sukzessive in das arterielle Gefäß 12 abgegeben werden. Alternativ oder zusätzlich kann auch eine primär diffusionsgesteuerte Freisetzung eines oder mehrerer Wirkstoffe aus dem biologisch abbaubaren Material erfolgen.

Fig. 10a zeigt einen Querschnitt des in einem arteriellen Gefäß 12 angeordneten Polymerkörpers 22, und in Fig. 10b ist ein Längsschnitt des Polymerkörpers 22 gezeigt. Der Polymerkörper 22 ist bevorzugt stromlinienförmig ausgebildet und weist ein proximales Ende 19 und ein distales Ende 20 auf. Der wirkstoffhaltige Polymerkörper 22 ist bevorzugt im Bereich seines proximalen Endes 19 durch Fixierflügel 23 in einer zentralen Position in dem Gefäß 12 gehalten. Die Fixierflügel 23 bewirken eine Selbstjustage des stromlinienförmigen Polymerkörpers 22 in einem zentralen Bereich des arteriellen Gefäßes 12. Die Fixierflügel 23 weisen eine kleine, bevorzugt linienförmig oder punktförmig ausgebildete Kontaktfläche 24 an der Wand 13 auf und sorgen somit für eine lumenerhaltende Aufhängung des implantierbaren Körpers 10 im Gefäß 12. Sind die Fixierflügel 23 mit Wirkstoff 11 beaufschlagt, findet aufgrund der kleinen Kontaktfläche 24 zur Wand 13 allenfalls ein geringer Wirkstoffeintrag in die Wand 13 statt. Alternativ können die Fixierflügel 23 auch wirkstofffrei ausgebildet sein.

## Patentansprüche

1. Implantierbare Vorrichtung zur lokalen Abgabe wenigstens eines Wirkstoffs (11) an vorgegebener Stelle in einen natürlichen Hohlraum (12) mit einer Wand (13) im Körper eines Säugers, **gekennzeichnet durch** wenigstens ein Wirkstoffreservoir (14), das in dem Hohlraum (12) fixierbar ist, wobei das Wirkstoffreservoir (14) und/oder ein Wirkstoffausgang (15) beabstandet zur Wand (13) so angeordnet ist, dass eine Freisetzung des wenigstens einen Wirkstoffs (11) entfernt zur Wand (13) erfolgt.

2. Implantierbare Vorrichtung zur lokalen Abgabe wenigstens eines Wirkstoffs (11) an vorgegebener Stelle in einen natürlichen Hohlraum (12) mit einer Wand (13) im Körper eines Säugers, **gekennzeichnet durch** wenigstens ein Wirkstoffreservoir (14) mit einem Wirkstoffausgang (15), wobei der Wirkstoffausgang (15) **durch** eine perforierte Hülle (16) einer spiralförmig oder schraubenförmig gewundenen Spule (17) gebildet ist.

3. Implantierbare Vorrichtung nach Anspruch 1 oder 2, **dadurch gekennzeichnet, dass** der Wirkstoffausgang (15) zentral im Hohlraum (12) angeordnet ist.

4. Implantierbare Vorrichtung nach wenigstens einem der vorhergehenden Ansprüche, **dadurch gekennzeichnet, dass** das wenigstens eine Wirkstoffreservoir (14) an wenigstens einem Ende (19, 20) ein Fixierelement (21) aufweist, das gegen die Hohlraumwände (13) gestützt ist.

5. Implantierbare Vorrichtung Anspruch 4, **dadurch gekennzeichnet, dass** das Fixierelement (21) den Wirkstoffausgang (15) bildet.

6. Implantierbare Vorrichtung nach einem der vorhergehenden Ansprüche 4 oder 5, **dadurch gekennzeichnet, dass** als Wirkstoffreservoir (14) das Fixierelement (21) ausgebildet ist.

7. Implantierbare Vorrichtung nach einem der vorhergehenden Ansprüche, **dadurch gekennzeichnet, dass** das Wirkstoffreservoir (14) aus mehreren Wirkstoffbehältern (25a, 25b, 25c) besteht.

8. Implantierbare Vorrichtung nach einem der vorhergehenden Ansprüche, **dadurch gekennzeichnet, dass** als Wirkstoffreservoir (14) die Spule (17) oder ein implantierbarer Stützkörper (Stent) (18) vorgesehen ist.

9. Implantierbare Vorrichtung nach einem der vorhergehenden Ansprüche, **dadurch gekennzeichnet, dass** das Wirkstoffreservoir (14) mit Hilfe des Fixierelements (21) koaxial fixiert ist.

10. Implantierbare Vorrichtung nach einem der vorhergehenden Ansprüche, **dadurch gekennzeichnet, dass** das Wirkstoffreservoir (14) aus der Spule (17) mit einem proximalen Ende (19) und einem distalen Ende (20) gebildet ist, wobei das proximale Ende (19) an der Innenwand (13) des Hohlraums (12) anliegt und die Spule (17) mit ihrem distalen Ende (20) etwa mittig im Hohlraum (12) angeordnet ist, und wobei das distale Ende (20) zur Abgabe des Wirkstoffs (11) vorgesehen ist.

11. Implantierbare Vorrichtung nach einem der vorhergehenden Ansprüche, **dadurch gekennzeichnet, dass** der wenigstens eine Wirkstoff (11) im Wirkstoffreservoir (14) durch Diffusion über Kapillarwirkung in den Hohlraum (12) freisetzbar ist.

12. Implantierbare Vorrichtung nach einem der vorhergehenden Ansprüche, **dadurch gekennzeichnet, dass** der wenigstens eine Wirkstoff (11) durch biologisch abbaubare Materialien in den Hohlraum (12) freisetzbar ist.

13. Implantierbare Vorrichtung nach einem der vorhergehenden Ansprüche, **dadurch gekennzeichnet, dass** das Wirkstoffreservoir (14) aus einem mehrschichtigen Körper aus vorzugsweise biologisch abbaubaren Materialien gebildet ist, wobei Räume zwischen den Schichten mit dem wenigstens einen Wirkstoff (11) gefüllt sind, und der Wirkstoff (11) nach Abbau der Schichten sukzessive in den Hohlraum (12) abgebbar ist.

14. Vorrichtung nach einem der vorhergehenden Ansprüche, **dadurch gekennzeichnet, dass** ein Polymerkörper (22) vorgesehen ist, der durch seine geometrische Form eine Selbstjustage des Wirkstoffreservoirs (14) in der Mitte des Hohlraums (12) bewirkt.

15. Vorrichtung nach Anspruch 14, **dadurch gekennzeichnet, dass** der Polymerkörper (22) wenigstens zwei Fixierflügel (23) aufweist, die eine kleine Kontaktfläche (24) zur Wand (13) des Hohlraums (12) aufweisen.
